(19) Europäisches Patentamt
European Patent Office
Office européen des brevets

(11) EP 4 506 453 A1

(12) EUROPEAN PATENT APPLICATION

(43) Date of publication:
**12.02.2025 Bulletin 2025/07**

(21) Application number: **24192481.0**

(22) Date of filing: **02.08.2024**

(51) International Patent Classification (IPC):
***C12N 9/12*** *(2006.01)*

(52) Cooperative Patent Classification (CPC):
**C12N 9/1252; C12Y 207/07007**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **04.08.2023 JP 2023128009**

(71) Applicant: **SYSMEX CORPORATION**
**Kobe-shi**
**Hyogo 651-0073 (JP)**

(72) Inventors:
- **OKAZAKI, Hironobu**
  **Kobe-shi, Hyogo 651-0073 (JP)**
- **ISHIKAWA, Tomokazu**
  **Kobe-shi, Hyogo 651-0073 (JP)**
- **KAWASAKI, Ryosuke**
  **Kobe-shi, Hyogo 651-0073 (JP)**
- **TAKEUCHI, Ryusuke**
  **Kobe-shi, Hyogo 651-0073 (JP)**
- **KANNO, Tatsuo**
  **Kobe-shi, Hyogo 651-0073 (JP)**
- **OIWA, Miki**
  **Kobe-shi, Hyogo 651-0073 (JP)**
- **SAKASHITA, Ryota**
  **Kobe-shi, Hyogo 651-0073 (JP)**

(74) Representative: **De Clercq & Partners**
**Edgard Gevaertdreef 10a**
**9830 Sint-Martens-Latem (BE)**

Remarks:
The complete document including Reference Table(s) and the Sequence Listing(s) can be downloaded from the EPO website

# (54) BST DNA POLYMERASE, REAGENT COMPOSITION, AND NUCLEIC ACID AMPLIFICATION METHOD

(57) Disclosed is a Bst DNA polymerase comprising: (1) an amino acid sequence set forth in SEQ ID NO: 1, or (2) an amino acid sequence comprising a consensus sequence Asn-X-(Ser/Thr) (X is any amino acid residue other than a proline residue), and comprising an amino acid sequence having 80% or more identity to the amino acid sequence set forth in SEQ ID NO: 1, and having 5' to 3' DNA polymerase activity and strand displacement activity, wherein the consensus sequence comprises an N-linked sugar chain, and the N-linked sugar chain comprises three or more mannose residues.

EP 4 506 453 A1

**Description**

FIELD OF THE INVENTION

The present invention relates to a Bst DNA polymerase having an N-linked sugar chain. The present invention relates to a reagent composition for use in a nucleic acid amplification method, containing the Bst DNA polymerase. The present invention relates to a nucleic acid amplification method using the Bst DNA polymerase having an N-linked sugar chain.

BACKGROUND

A Bst DNA polymerase is a heat-resistant DNA polymerase named after the the organism (Geobacillus stearothermophilus - former Bacillus stearothermophilus) from which it was originally isolated. The Bst DNA polymerase has 5' to 3' DNA polymerase activity and strand displacement activity. Since the Bst DNA polymerase has strand displacement activity, the Bst DNA polymerase can perform an isothermal nucleic acid amplification reaction. The isothermal nucleic acid amplification reaction is used for, for example, genetic testing for cancer, genetic testing for detecting viruses or bacteria, and the like in fields such as medical care, food hygiene, and environmental hygiene. Since the activity of Bst DNA polymerase is maximized at 60°C to 65°C, the Bst DNA polymerase is also suitable for synthesis of DNA strands having a high GC content.

U.S. Patent No. 5,814,506 describes that a recombinant Bst DNA polymerase is prepared by an E. coli expression system.

SUMMARY OF THE INVENTION

In general, in a nucleic acid amplification reaction, it is known that amplification efficiency of a target nucleic acid is reduced by mixing of interfering substances into a reaction solution. The interfering substances are substances that cause inhibition of a nucleic acid amplification reaction, a decrease in reaction rate, and the like. The interfering substances may be contained in a sample containing the target nucleic acid, for example, a biological sample such as blood, cells, or fixed embedded tissue. A reagent used for extracting and purifying the target nucleic acid from such a sample can also be one of interfering substances. In the nucleic acid amplification reaction, it is known that non-specific amplification that does not depend on a nucleotide sequence of the target nucleic acid easily occurs at a temperature lower than the optimal reaction temperature of DNA polymerase to be used. For example, when the reaction solution is heated to the optimal reaction temperature, a non-specific amplification product may be generated before the reaction solution reaches the optimal reaction temperature. When the isothermal nucleic acid amplification reaction using Bst DNA polymerase is used for detection of a target nucleic acid, the influence of interfering substances and reactivity at a temperature lower than the optimal reaction temperature may reduce detection sensitivity.

An object of the present invention is to provide a Bst DNA polymerase capable of reducing the influence of interfering substances and reactivity at a temperature lower than the optimal reaction temperature. An object of the present invention is to provide a reagent composition containing the Bst DNA polymerase and a nucleic acid amplification method using the Bst DNA polymerase.

The present inventors had found that when isothermal nucleic acid amplification was performed using the Bst DNA polymerase prepared by the present inventors, the influence of interfering substances and reactivity at a temperature lower than the optimal reaction temperature were reduced as compared with the case of using the conventional Bst DNA polymerase, and thus the present invention was completed. The present invention provides the following [1] to [21].

[1] A Bst DNA polymerase containing: (1) an amino acid sequence set forth in SEQ ID NO: 1, or (2) an amino acid sequence containing a consensus sequence Asn-X-(Ser/Thr) (X is any amino acid residue other than a proline residue), and containing an amino acid sequence having 80% or more identity to the amino acid sequence set forth in SEQ ID NO: 1, and having 5' to 3' DNA polymerase activity and strand displacement activity, in which the consensus sequence contains an N-linked sugar chain, and the N-linked sugar chain contains three or more mannose residues.
[2] The Bst DNA polymerase according to [1], containing the consensus sequence in a finger domain.
[3] The Bst DNA polymerase according to [1] or [2], in which the N-linked sugar chain contains nine or less mannose residues.
[4] The Bst DNA polymerase according to any one of [1] to [3], in which the N-linked sugar chain has a structure represented by the following formula (I):

[Chemical Formula 1]

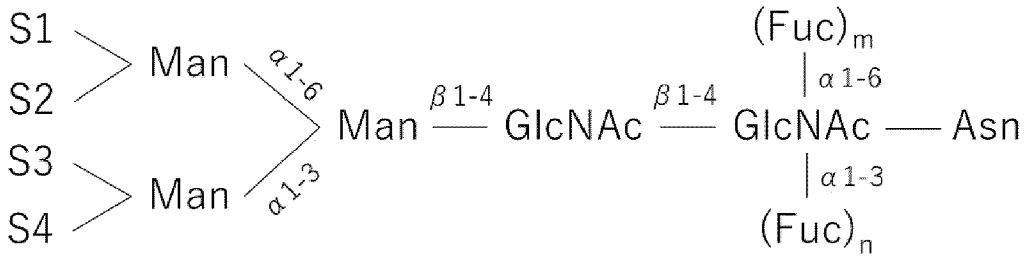

(I)

wherein Asn is an asparagine residue in the consensus sequence, Man is a mannose residue, GlcNAc is an N-acetyl D-glucosamine residue, Fuc is a fucose residue, m and n are each independently 0 or 1, and S1, S2, S3, and S4 are each independently absent or a sugar chain containing one or more and five or less monosaccharide residues.

[5] The Bst DNA polymerase according to [4], in which the S1 contains at least one monosaccharide residue selected from the group consisting of a mannose residue, an N-acetyl D-glucosamine residue, and a glucose residue.

[6] The Bst DNA polymerase according to [4] or [5], in which the S2 contains at least one monosaccharide residue selected from the group consisting of a mannose residue, an N-acetyl D-glucosamine residue, and a glucose residue.

[7] The Bst DNA polymerase according to any one of [4] to [6], in which the S3 contains at least one monosaccharide residue selected from the group consisting of a mannose residue, an N-acetyl D-glucosamine residue, and a glucose residue.

[8] The Bst DNA polymerase according to any one of [4] to [7], in which the S4 contains at least one monosaccharide residue selected from the group consisting of a mannose residue, an N-acetyl D-glucosamine residue, and a glucose residue.

[9] The Bst DNA polymerase according to [4], in which the S1, the S2, the S3, and the S4 are absent.

[10] The Bst DNA polymerase according to any one of [4] to [8], in which at least one sugar chain of the S1, the S2, the S3, and the S4 is an N-acetyl D-glucosamine residue.

[11] The Bst DNA polymerase according to any one of [4] to [8], in which at least one sugar chain of the S1, the S2, the S3, and the S4 has a structure represented by the following formula (II):

[Chemical Formula 2]

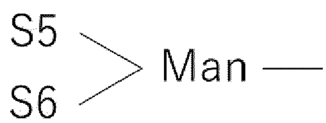

(II)

wherein S5 and S6 are each independently absent or a sugar chain containing one or more and three or less mannose residues.

[12] The Bst DNA polymerase according to any one of [1] to [11], in which at least one non-reducing end of the N-linked sugar chain is a mannose residue or an N-acetyl D-glucosamine residue.

[13] The Bst DNA polymerase according to [1] to [12], in which the m is 1 and the n is 0, or the m is 0 and the n is 1.

[14] A reagent composition for use in a nucleic acid amplification method, containing the Bst DNA polymerase according to any one of [1] to [13].

[15] The reagent composition according to [14], in which the nucleic acid amplification method is performed under substantially isothermal conditions.

[16] The reagent composition according to [14] or [15], wherein the nucleic acid amplification method is a LAMP method, an RCA method, a NASBA method, an SDA method, a TRC method, or an ICAN method.

[17] A reagent kit for use in a nucleic acid amplification method, including the reagent composition according to any one of [14] to [16] and dNTPs.

[18] The reagent kit according to [17], in which the nucleic acid amplification method is performed under substantially isothermal conditions.

[19] The reagent kit according to [17] or [18], in which the nucleic acid amplification method is a LAMP method, an RCA method, a NASBA method, an SDA method, a TRC method, or an ICAN method.

[20] A nucleic acid amplification method including amplifying a target nucleic acid using the Bst DNA polymerase according to any one of [1] to [13].

[21] The nucleic acid amplification method according to [20], including performing an amplification reaction of the target nucleic acid under substantially isothermal conditions.

According to the present invention, it is possible to provide a Bst DNA polymerase capable of reducing the influence of interfering substances and reactivity at a temperature lower than the optimal reaction temperature. According to the present invention, a reagent composition containing the Bst DNA polymerase and a nucleic acid amplification method using the Bst DNA polymerase can be provided.

BRIEF DESCRIPTION OF THE DRAWINGS

Fig. 1 is a diagram showing an example of an appearance of a reagent kit.
Fig. 2 is a diagram showing an example of an appearance of a reagent kit.
Fig. 3 is a diagram showing results of lectin blots of polymerase of Example 1 and polymerase of Comparative Example.
Fig. 4 is a graph showing a difference in nucleic acid amplification change rate between the polymerase of Comparative Example and the polymerase of Example 1 in the presence of each of interfering substances.
Fig. 5 is a graph showing the nucleic acid amplification change rate of the polymerase of Comparative Example and the polymerase of Example 1 at each temperature.

DETAILED DESCRIPTION OF THE PREFERRED EMBODIMENTS

The Bst DNA polymerase of the present invention (hereinafter, also referred to as "the polymerase of the present invention") is a DNA polymerase characterized by having an N-linked sugar chain containing three or more mannose residues in a consensus sequence in the amino acid sequence. The consensus sequence in the amino acid sequence is a sequence containing an asparagine residue (Asn) to which an N-linked sugar chain is bound. The consensus sequence consists of three amino acid residues of Asn-X-(Ser/Thr). The "X" is any amino acid residue other than a proline residue. The "Ser/Thr" indicates a serine residue or a threonine residue. The consensus sequence is NX(S/T) in one-letter notation. As used herein, the term "Bst DNA polymerase" is also used to refer to a mixture of Bst DNA polymerases having the same amino acid sequence but varying N-linked sugar chains as will be detailed herein. The nature of the sugar chain is described herein below.

The polymerase of the present invention has 5' to 3' DNA polymerase activity and strand displacement activity as enzyme activity. The 5' to 3' DNA polymerase activity refers to an activity in which a polymerase synthesizes a DNA strand complementary to single-stranded DNA serving as a template in a direction from 5' to 3'. The strand displacement activity refers to, when there is a region that is double-stranded DNA in the extending direction in the process of synthesizing a DNA strand complementary to template DNA, an activity in which a polymerase synthesizes a complementary DNA strand while the polymerase dissociates hydrogen bonds of double-stranded DNA. Since the polymerase of the present invention has strand displacement activity, heat treatment at a high temperature (for example, 90°C to 99°C) for dissociating double-stranded DNA as a template is unnecessary in a nucleic acid amplification reaction. That is, the polymerase of the present invention can perform an amplification reaction using double-stranded DNA as a template under substantially isothermal conditions.

In the present specification, the term "substantially isothermal" refers to a substantially constant reaction temperature. Since the optimal reaction temperature of the polymerase of the present invention is about 60°C to 65°C, the reaction temperature is preferably 55°C to 70°C, more preferably 58°C to 67°C, and still more preferably 60°C to 65°C. The amplification reaction under substantially isothermal conditions can be performed, for example, by incubation using a device such as an incubator or a thermal cycler set to have a constant temperature. In the case of performing incubation with the device, after the temperature is raised to the above reaction temperature, the amplification reaction can be performed by setting the temperature to be kept constant within the above range of the optimal reaction temperature. The temperature during the amplification reaction does not need to be completely constant. The temperature during the amplification reaction may vary within the above range of the optimal reaction temperature.

The polymerase of the present invention may be a protein containing the amino acid sequence set forth in SEQ ID NO: 1. The protein containing the amino acid sequence set forth in SEQ ID NO: 1 can be, for example, a protein consisting of an amino acid sequence in which one or more amino acid residues are added at the N-terminal and/or C-terminal of the amino acid sequence set forth in SEQ ID NO: 1. When a plurality of amino acid residues is added, the plurality of amino acid residues can constitute, for example, a tag peptide described later. Alternatively, the polymerase of the present invention may be a protein consisting of the amino acid sequence set forth in SEQ ID NO: 1. The amino acid sequence set forth in SEQ ID NO: 1 is as follows. In the sequence, the underlined part indicates the consensus sequence.

AEGEKPLEEMEFAIVDVITEEMLADKAALVVEVMEENYHDAPIVGIALVNEHG
RFFMRPETALADSQFLAWLADETKKKSMFDAKRAVVALKWKGIELRGVAFDL
LLAAYLLNPAQDAGDIAAVAKMKQYEAVRSDEAVYGKGVKRSLPDEQTLAEH
LVRKAAAIWALEQPFMDDLRNNEQDQLLTKLEQPLAAILAEMEFTGVNVDTK
RLEQMGSELAEQLRAIEQRIYELAGQEFNINSPKQLGVILFEKLQLPVLKKTKTG
YSTSADVLEKLAPHHEIVENILHYRQLGKLQSTYIEGLLKVVRPDTGKVHTMFN
QALTQTGRLSSAEPNLQNIPIRLEEGRKIRQAFVPSEPDWLIFAADYSQIELRVLA
HIADDDNLIEAFQRDLDIHTKTAMDIFHVSEEEVTANMRRQAKAVNFGIVYGIS
DYGLAQNLNITRKEAAEFIERYFASFPGVKQYMENIVQEAKQKGYVTTLLHRR
RYLPDITSRNFNVRSFAERTAMNTPIQGSAADIIKKAMIDLAARLKEEQLQARLL
LQVHDELILEAPKEEIERLCELVPEVMEQAVTLRVPLKVDYHYGPTWYDAK

(SEQ ID NO: 1)

The amino acid sequence set forth in SEQ ID NO: 1 corresponds to a portion from the 290th to 876th of the amino acid sequence (SEQ ID NO: 2) of the full-length protein encoded by the Bst DNA polymerase gene of Geobacillus stearothermophilus. The amino acid sequence set forth in SEQ ID NO: 1 is also referred to as a large fragment of a full-length protein. The amino acid sequence set forth in SEQ ID NO: 1 lacks the 5' to 3' exonuclease domain of the full-length protein. Therefore, the Bst DNA polymerase containing the amino acid sequence set forth in SEQ ID NO: 1 does not have 5' to 3' exonuclease activity. The amino acid sequence set forth in SEQ ID NO: 2 is as follows. In the sequence, the underlined part is the amino acid sequence set forth in SEQ ID NO: 1.

MKKKLVLIDGNSVAYRAFFALPLLHNDKGIHTNAVYGFTMMLNKILAEEQPTH

LLVAFDAGKTTFRHETFQEYKGGRQQTPPELSEQFPLLRELLKAYRIPAYELDH
YEADDIIGTLAARAEQEGFEVKIISGDRDLTQLASRHVTVDITKKGITDIEPYTPE
TVREKYGLTPEQIVDLKGLMGDKSDNIPGVPGIGEKTAVKLLKQFGTVENVLAS
IDEVKGEKLKENLRQHRDLALLSKQLASICRDAPVELSLDDIVYEGQDREKVIA
LFKELGFQSFLEKMAAPAAEGEKPLEEMEFAIVDVITEEMLADKAALVVEVME
ENYHDAPIVGIALVNEHGRFFMRPETALADSQFLAWLADETKKKSMFDAKRA
VVALKWKGIELRGVAFDLLLAAYLLNPAQDAGDIAAVAKMKQYEAVRSDEA
VYGKGVKRSLPDEQTLAEHLVRKAAAIWALEQPFMDDLRNNEQDQLLTKLEQ
PLAAILAEMEFTGVNVDTKRLEQMGSELAEQLRAIEQRIYELAGQEFNINSPKQ
LGVILFEKLQLPVLKKTKTGYSTSADVLEKLAPHHEIVENILHYRQLGKLQSTYI
EGLLKVVRPDTGKVHTMFNQALTQTGRLSSAEPNLQNIPIRLEEGRKIRQAFVPS
EPDWLIFAADYSQIELRVLAHIADDDNLIEAFQRDLDIHTKTAMDIFHVSEEEVT
ANMRRQAKAVNFGIVYGISDYGLAQNLNITRKEAAEFIERYFASFPGVKQYME
NIVQEAKQKGYVTTLLHRRRYLPDITSRNFNVRSFAERTAMNTPIQGSAADIIKK
AMIDLAARLKEEQLQARLLLQVHDELILEAPKEEIERLCELVPEVMEQAVTLRV
PLKVDYHYGPTWYDAK (SEQ ID NO: 2)

The polymerase of the present invention may be a protein containing an amino acid sequence having 80% or more identity to the amino acid sequence set forth in SEQ ID NO: 1 as long as the polymerase contains the consensus sequence Asn-X-(Ser/Thr) and has the N-linked sugar chain and the enzyme activity. Preferably, the polymerase of the present invention may be a protein containing an amino acid sequence having 85% or more, 90% or more, 95% or more, 96% or more, 97% or more, 98% or more, or 99% or more identity to the amino acid sequence set forth in SEQ ID NO: 1. Alternatively, the polymerase of the present invention may be a protein consisting of an amino acid sequence having 80% or more, 85% or more, 90% or more, 95% or more, 96% or more, 97% or more, 98% or more, or 99% or more identity to the amino acid sequence set forth in SEQ ID NO: 1. The identity of the amino acid sequence can be determined by sequence alignment. Sequence alignment itself is known. Sequence alignment refers to a process of aligning two amino acid sequences to be compared and detecting amino acid residues matched between these amino acid sequences. Sequence alignment indicates the number of identical amino acid residues between two amino acid sequences in percentage. Sequence alignment can be performed by, for example, a known multiple alignment program such as ClustalW, TREBMAL, or BLAST, commercially available protein analysis software such as Discovery Studio, or the like.

The amino acid sequence having 80% or more identity to the amino acid sequence set forth in SEQ ID NO: 1 may be an amino acid sequence including modification of one or more amino acid residues with respect to the amino acid sequence set forth in SEQ ID NO: 1. The modification of amino acid residue(s) refers to substitution, addition and deletion of amino acid residues, and combinations thereof. The modification of amino acid residue(s) in the amino acid sequence set forth in SEQ ID NO: 1 may be, for example, conservative substitution of amino acid residue(s). The conservative substitution refers to substitution of one amino acid residue in a sequence with another amino acid residue having similar properties such as structure, charge, polarity, or hydrophobicity. Examples thereof include a substitution between a phenylalanine residue, a tryptophan residue and a tyrosine residue, a substitution between a serine residue and a threonine residue, a substitution between a lysine residue, an arginine residue and a histidine residue, and a substitution

between an aspartic acid residue and a glutamic acid residue. Alternatively, the modification of amino acid residue(s) in the amino acid sequence set forth in SEQ ID NO: 1 may be a modification corresponding to a mutation of a known mutant of Bst DNA polymerase. The mutants of Bst DNA polymerase are described, for example, in Ma Y. et al., BioMed Research International, (2016) vol. 2016: 2906484 and Paik I. et al., Biochemistry, (2023) vol. 62, pp. 410-418.

For example, the polymerase of the present invention may have at least one modification selected from the group consisting of deletion of the first alanine residue (A1), substitution of the 311st glycine residue (G311), substitution of the 366th serine residue (S366), substitution of the 413rd arginine residue (R413), substitution of the 417th lysine residue (K417), substitution of the 438th isoleucine residue (I438), substitution of the 439th threonine residue (T439), substitution of the 488th threonine residue (T488), substitution of the 541st aspartic acid residue (D541), and substitution of the 547th alanine residue (A547) in the amino acid sequence set forth in SEQ ID NO: 1.

In the amino acid sequence set forth in SEQ ID NO: 1, examples of the substitution of G311 include substitution with an alanine residue (G311A) and substitution with a leucine residue (G311L). Examples of the substitution of S366 include substitution with an aspartic acid residue (S366D). Examples of the substitution of R413 include substitution with an alanine residue (R413A) and substitution with a glutamic acid residue (R413E). Examples of the substitution of K417 include substitution with an alanine residue (K417A) and substitution with an aspartic acid residue (K417D). Substitutions of I438 and T439 are substitutions of the consensus sequence. That is, the substitution of I438 is substitution with an arbitrary amino acid residue other than a proline residue, and the substitution of T439 is substitution with a serine residue (T439S). Examples of the substitution of T488 include substitution with an asparagine residue (T488N). Examples of the substitution of D541 include substitution with an alanine residue (D541A) and substitution with a glutamic acid residue (D541E). Examples of the substitution of A547 include substitution with a glycine residue (A547G).

In general, DNA polymerase is known to have a highly conserved structure. Specifically, the three-dimensional structure of the DNA polymerase resembles the right hand of a human, and the DNA polymerase includes, from the N-terminal side, a thumb domain corresponding to a thumb, a palm domain corresponding to a palm, and a finger domain corresponding to fingers. The DNA polymerase binds to the template DNA so as to grip with these three domains. The polymerase of the present invention preferably includes a thumb domain, a palm domain, and a finger domain. In the amino acid sequence set forth in SEQ ID NO: 1, a consensus sequence Asn-Ile-Thr is known to be included in a finger domain. The polymerase of the present invention preferably includes the consensus sequence Asn-X-(Ser/Thr) in the finger domain. The finger domain of the polymerase of the present invention can be confirmed, for example, by examining the three-dimensional structure of the polymerase of the present invention with commercially available protein analysis software such as Discovery Studio.

As described above, the polymerase of the present invention has an N-linked sugar chain in the consensus sequence present in the amino acid sequence. More specifically, the N-linked sugar chain is covalently bonded to a nitrogen atom in a side chain of the asparagine residue in the consensus sequence. The number of N-linked sugar chains in the polymerase of the present invention may be one or more. The Bst DNA polymerase consisting of the amino acid sequence of SEQ ID NO: 1 has one consensus sequence, and thus has one N-linked sugar chain. When the polymerase of the present invention has a plurality of consensus sequences, the polymerase may have one or more N-linked sugar chains.

The N-linked sugar chain contains three or more mannose residues. More specifically, the N-linked sugar chain contains at least a core structure represented by the following formula (III). Hereinafter, "Asn" in the formula indicates the asparagine residue in the consensus sequence. As represented by the formula (III), the core structure is composed of three mannose residues (Man) and two N-acetyl D-glucosamine residues (GlcNAc).

[Chemical Formula 3]

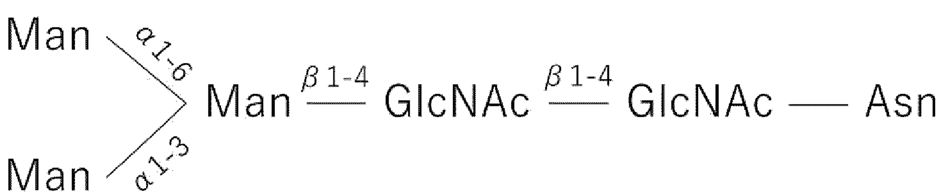

(III)

The N-linked sugar chain is added to the consensus sequence of Bst DNA polymerase by preparing the Bst DNA polymerase by an in vitro expression system of a recombinant protein using a host capable of modifying the sugar chain of the protein. Such a host is preferably a eukaryote, and examples thereof include yeast, insect cells, mammalian cells, insects, and the like. Among them, insects and insect cells are preferable, and lepidopteran insects and insect cells thereof are particularly preferable. Lepidopteran insects suitable for an expression system of a recombinant protein are known, and examples thereof include Bombyx mori, Spilosoma imparilis, Antheraea pernyi, Spodoptera frugiperda, Trichoplusiani, and the like.

Among them, Bombyx mori is particularly preferable. The lepidopteran insect may be any of an adult, a pupa, and a larva, but it is preferable to use a pupa from the viewpoint of serine protease activity and sensitivity to a baculovirus. The cell of the lepidopteran insect is not particularly limited as long as it is a cell line established from the above lepidopteran insect, and examples thereof include BmN, BmN4, Splm, Anpe, Sf9, Sf21, High5, and the like.

The N-linked sugar chain can be added to Bst DNA polymerase by, for example, a protein expression system using Bombyx mori (hereinafter, also referred to as "Bombyx mori expression system") as a host. Generally, in the endoplasmic reticulum of Bombyx mori, a protein containing an N-linked sugar chain to which up to nine mannose residues are added can be generated. In a Golgi apparatus, stepwise cleavage of the added mannose residue, addition and cleavage of an N-acetyl D-glucosamine residue to the non-reducing end, addition of one or two fucose residues to the core structure, addition of a glucose residue to the non-reducing end, and the like can be performed to form N-linked sugar chains having various structures. Preferably, at least one non-reducing end of the N-linked sugar chain is a mannose residue or an N-acetyl D-glucosamine residue. Accordingly, the invention also provides a Bst DNA polymerase obtainable by expressing a sequence encoding a Bst DNA polymerase in a Bombyx mori expression system. Preferably said Bst DNA polymerase represents a mixture of Bst DNA polymerases having varying types of N-linked sugar chains as described below.

The N-linked sugar chain that can be contained in the polymerase of the present invention prepared by the Bombyx mori expression system is, for example, a sugar chain having a structure represented by the formula (I) (hereinafter, also referred to as a "sugar chain of formula (I)"). In the Bombyx mori expression system, a plurality of molecules corresponding to the polymerase of the present invention can be prepared. The structure of the sugar chain of the formula (I) in the plurality of molecules corresponding to the polymerase of the present invention is not limited to one type. That is, the plurality of molecules of the polymerase of the present invention may contain two or more types of sugar chains of the formula (I). Examples of the sugar chain of the formula (I) include sugar chains having structures represented by the following (IV) to (XI).

[Chemical Formula 4]

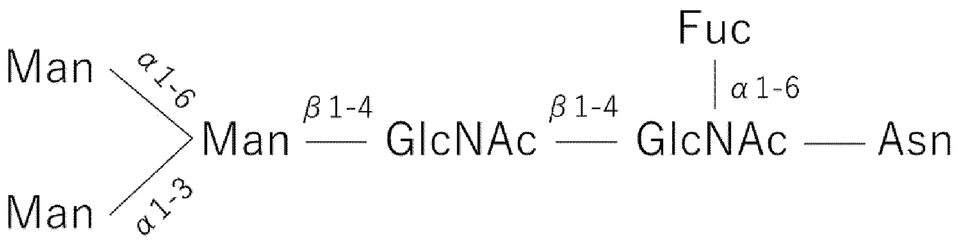

(IV)

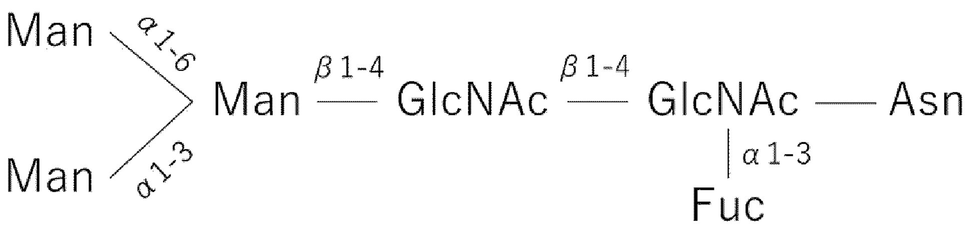

(V)

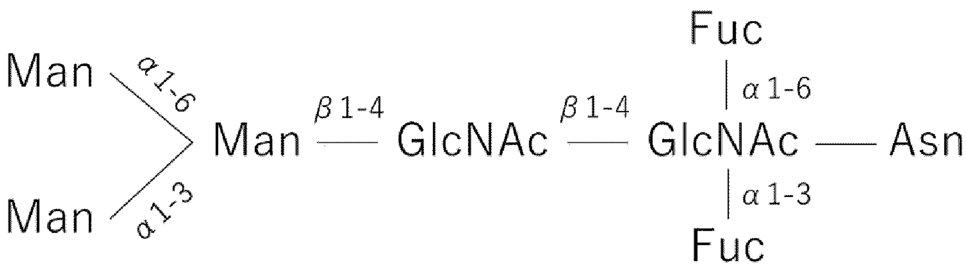

(VI)

[Chemical Formula 5]

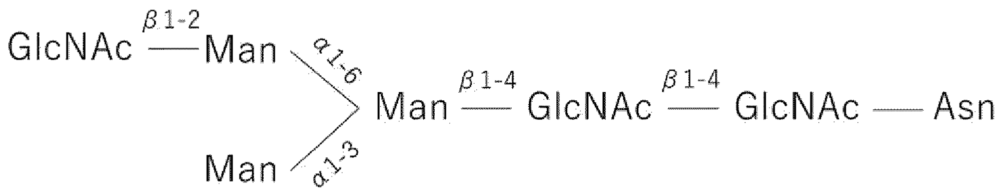

and/or

(VII)

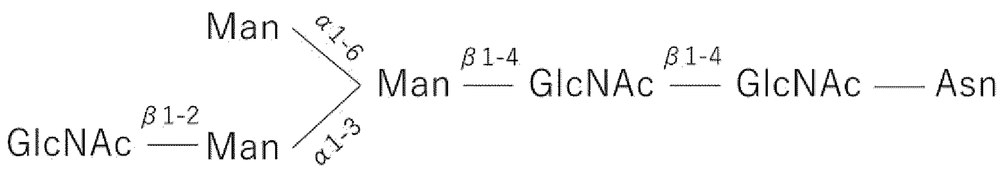

[Chemical Formula 6]

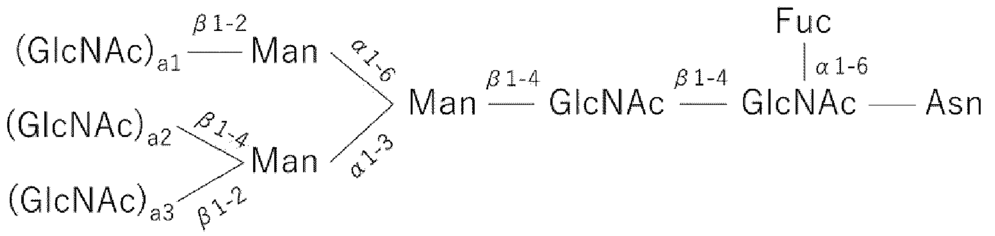

(VIII)

wherein a1, a2, and a3 are each independently an integer of 0 or more and 2 or less, and the sum of a1, a2, and a3 is 1 or more and 3 or less

[Chemical Formula 7]

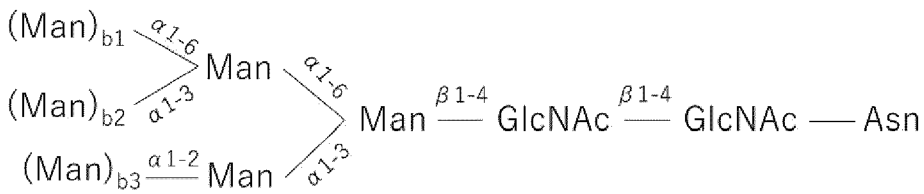

(IX)

wherein b1, b2, and b3 are each independently an integer of 0 or more and 2 or less, and the sum of b1, b2, and b3 is 4 or less

[Chemical Formula 8]

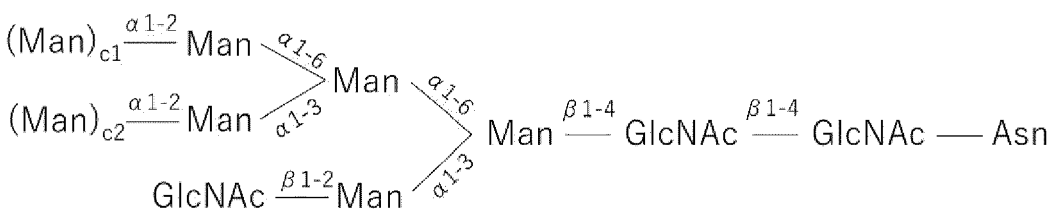

(X)

wherein c1 and c2 are each independently an integer of 0 or more and 2 or less

[Chemical Formula 9]

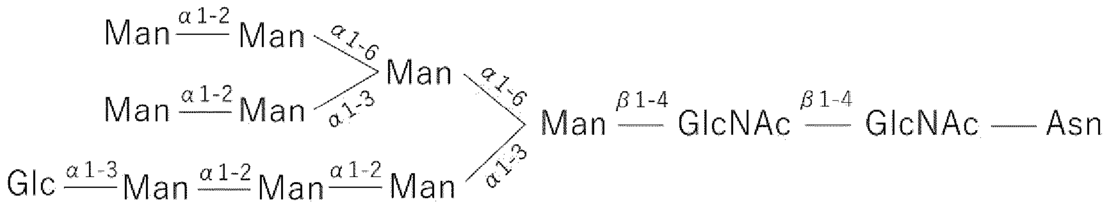

(XI)

The N-linked sugar chains having the structures represented by the above formulas (IV) to (VI) correspond to a case in which S1, S2, S3, and S4 are absent in the formula (I). The N-linked sugar chain having the structure represented by the formula (IV) corresponds to a case in which S1, S2, S3, and S4 are not present, m is 1, and n is 0 in the formula (I). The N-linked sugar chain having the structure represented by the formula (V) corresponds to a case in which S 1, S2, S3, and S4 are not present, m is 0, and n is 1 in the formula (I). The N-linked sugar chains having the structures represented by the above formulas (VII), (VIII), and (X) correspond to a case in which at least one of the sugar chains of S 1, S2, S3, and S4 in the formula (I) is an N-acetyl D-glucosamine residue.

In the formula (I), when at least one of the sugar chains S1, S2, S3, and S4 has the structure represented by the formula (II), examples of the sugar chain of the formula (I) include sugar chains having structures represented by the following (XII) to (XIV).

[Chemical Formula 10]

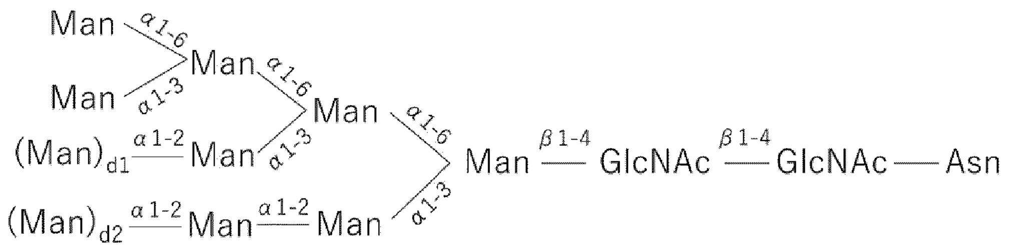

(XII)

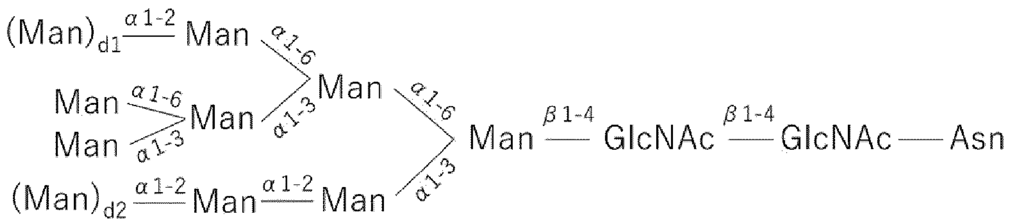

(XIII)

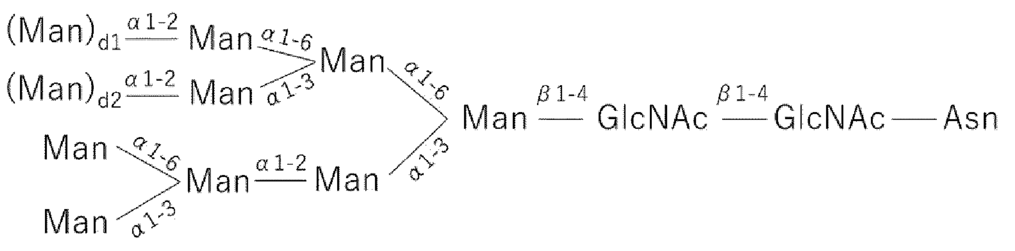

(XIV)

wherein d1 and d2 are each independently 0 or 1, and the sum of d1 and d2 is 0 or 1.

As described above, the polymerase of the present invention can be prepared by an in vitro expression system of a recombinant protein using a host capable of modifying a sugar chain of a protein. Methods for making a recombinant protein using such an expression system are known per se. For example, when the host is yeast or cultured cells, the polymerase of the present invention can be expressed by transfecting yeast or cultured cells with vector DNA containing a gene encoding the polymerase of the present invention by a known gene transfer method. The polymerase of the present invention can be obtained by solubilizing yeast or cultured cells expressing the polymerase of the present invention with a

solution containing a cytolytic agent such as a surfactant.

In a preferred embodiment, the baculovirus recombined with vector DNA is used to infect a lepidopteran insect or cultured cells of the insect to express the polymerase of the present invention. When a lepidopteran insect is used, the polymerase of the present invention can be obtained by collecting a body fluid or disrupting the insect to prepare a homogenate. If necessary, the polymerase of the present invention may be separated and purified from the homogenate by a known method such as centrifugation, salting-out, or chromatography. When cultured cells of the lepidopteran insect are used, the polymerase of the present invention can be obtained in the same manner as when yeast or cultured cells are used. Since the polymerase of the present invention is a heat-resistant DNA polymerase, the baculovirus may be inactivated by heating a solution containing the polymerase of the present invention to 60°C to 70°C.

When the polymerase of the present invention is a Bst DNA polymerase containing the amino acid sequence of SEQ ID NO: 1, the gene or DNA sequence encoding the polymerase has, for example, a nucleotide sequence shown in SEQ ID NO: 3.

The vector DNA may be DNA having a promoter that enables gene expression in vitro in a host capable of sugar chain modification of a protein, and capable of inserting a gene or DNA sequence encoding the polymerase of the present invention downstream of the promoter. Such vector DNA itself is known, and vector DNA for protein expression in a host is commercially available. If necessary, a DNA sequence encoding a tag peptide may be inserted upstream or downstream of the DNA sequence encoding the polymerase of the present invention. Thereby, the polymerase of the present invention fused with the tag peptide can be obtained. The tag peptide is preferably a tag for protein purification, and examples thereof include a His tag, a glutathione-S-transferase (GST) tag, a maltose binding protein (MBP) tag, and the like.

A preferred vector DNA is a transfer vector capable of preparing a recombinant baculovirus into which the above gene is inserted by homologous recombination with baculovirus DNA. Such a transfer vector itself is known and examples thereof include pM02, pYNG, pBM030, pBM050, pVL1392, and the like. The promoter can be appropriately selected from known promoters such as a polyhedrin promoter, a p10 promoter, and a Bombyx mori actin promoter. A method for recombining baculovirus with DNA having a desired nucleotide sequence is known per se. For example, first, baculovirus DNA linearized by a restriction enzyme or the like and a transfer vector into which a polynucleotide sequence or gene encoding the polymerase of the present invention is incorporated are co-transfected into cultured cells of lepidopteran insects. Then, by screening infected cells, a recombinant baculovirus can be obtained.

The type of baculovirus is not particularly limited as long as the baculovirus is a virus capable of infecting the above lepidopteran insect or insect cells thereof. A preferred baculovirus is a nuclear polyhedrosis virus (NPV) or a modified virus thereof. Examples of such a virus include BmNPV, HycuNPV, AnpeNPV, AcNPV, and a recombinant baculovirus that infects a host such as Bombyx mori of Bombycidae or Autographa californica of Noctuidae (see Japanese Laid-Open Patent Publication No. 2003-052371). Preferably, a cysteine protease-deficient (CPd) baculovirus is used (see Japanese Patent Application No. H07-303488).

Means for infecting a lepidopteran insect or insect cells thereof with the recombinant baculovirus can be appropriately selected from known methods, without particular restriction. For example, when the lepidopteran insect is infected, a method of injecting a liquid containing the recombinant baculovirus into the insect can be envisaged. When the cultured cells are infected, a liquid containing the recombinant baculovirus may be added to a culture medium. Five days to eight days after the lepidopteran insect or insect cells are infected with the virus, the polymerase of the present invention is expressed in the lepidopteran insect or insect cells thereof.

The present invention also includes a reagent composition for use in a nucleic acid amplification method, containing one or more of the polymerase of the present invention (hereinafter, also referred to as "the reagent composition of the present invention"). The reagent composition of the present invention may contain a component for stably preserving the one or more polymerases. Examples of such components include buffers (for example, Tris-HCl), antifreeze (for example, glycerol), inorganic salts (KCl, NaCl, and the like), reducing agents (for example, dithiothreitol (DTT)), chelating agents (for example, ethylenediaminetetraacetic acid (EDTA)), surfactants (for example, Triton (trademark) X-100), and the like. The reagent composition of the present invention may be in a solid (for example, powder) state or in a solution state. The reagent composition of the present invention may be stored in a container and provided to a user. The reagent composition of the present invention stored in the container may be further packaged in a box and provided to a user.

The reagent composition of the present invention can be used in a nucleic acid amplification method performed under substantially isothermal conditions. The substantially isothermal conditions are as described above. The nucleic acid amplification method performed under substantially isothermal conditions is known, and examples thereof include a loop-mediated isothermal amplification (LAMP) method, a rolling circle amplification (RCA) method, a nucleic acid sequence-based amplification (NASBA) method, a strand displacement amplification (SDA) method, a transcription reverse-transcription concerted reaction (TRC) method, an isothermal and chimeric primer-initiated amplification of nucleic acids (ICAN) method, and the like. For example, see U.S. Patent. No. 6410278 for the LAMP method, U.S. Patent No. 6,291,187 for the RCA method, U.S. Patent No. 5554517for the NASBA method, U.S. Patent No. 5,270,184 for the SDA method, U.S. Patent Application Publication No. 20010053518 for the TRC method, and European Patent No.

1167524for the ICAN method.

The present invention also includes a reagent kit for use in a nucleic acid amplification method. The reagent kit includes one or more polymerases or the reagent composition of the present invention. The reagent composition of the present invention is as described above. The reagent kit may further contain dNTPs and/or a buffer solution. The "dNTPs" is a mixture containing deoxyadenosine triphosphate (dATP), deoxycytidine triphosphate (dCTP), deoxyguanosine triphosphate (dGTP), and deoxythymidine triphosphate (dTTP). The dNTPs are usually provided in a solution or a frozen product thereof. The reagent kit can be used in a nucleic acid amplification method performed under substantially isothermal conditions. The nucleic acid amplification method performed under substantially isothermal conditions is as described later.

Fig. 1 shows an example of the reagent kit. In Fig. 1, 10 denotes a reagent kit, 11 denotes a first container containing the reagent composition of the present invention, 12 denotes a packing box, and 13 denotes an attached document. A method of using the reagent composition, a method of storing the reagent composition, and the like may be described in the attached document.

Fig. 2 shows another example of the reagent kit. In Fig. 2, 20 denotes a reagent kit, 21 denotes a first container containing the reagent composition of the present invention, 22 denotes a second container containing dNTPs, 23 denotes a packing box, and 24 denotes an attached document. A method of using the various reagents, a method of storing the various reagents, and the like may be described in the attached document.

The present invention also includes a nucleic acid amplification method including amplifying a target nucleic acid using the polymerase of the present invention (hereinafter, also referred to as "the method of the present invention"). The target nucleic acid is any nucleic acid desired to be amplified. Preferably, the target nucleic acid is single-stranded or double-stranded DNA. The target nucleic acid may be cDNA generated from RNA by a reverse transcription reaction.

The origin of the target nucleic acid is not particularly limited. The target nucleic acid may be a viral, prokaryotic, or eukaryotic nucleic acid. Examples of the virus include coronavirus (such as MERS-CoV, SARS-CoV, and SARS-CoV-2), influenza virus, poxvirus, norovirus, RS virus, adenovirus, human immunodeficiency virus, human papilloma virus, hepatitis virus, and the like. Examples of the prokaryote include Escherichia coli, Salmonella, Pseudomonas aeruginosa, Staphylococcus aureus, Campylobacter, Listeria, legionella, Mycobacterium tuberculosis, Mycoplasma, Pertussis, and the like. Examples of the eukaryote include Cryptosporidium, Giardia, malaria parasites, fungi, birds, mammals, and the like. A preferred mammal is a human. The prokaryotic and eukaryotic nucleic acids may be nucleic acids of cultured cells. In the method of the present invention, a sample containing the nucleic acid is subjected to amplification. In the case of a sample containing RNA, the sample after a reverse transcription reaction is subjected to amplification. Examples of the sample to be subjected to amplification include a sample or excrement collected from a living body, a washing liquid of a living body, a food product, a solubilized product thereof, an extract thereof, and the like. Examples of the sample collected from a living body include an organ, a tissue, a cell, a body fluid, sputum, and the like. Examples of the body fluid include blood, plasma, serum, lymph, saliva, and the like. Examples of the excrement include urine and feces. Examples of the washing liquid of a living body include body cavity washing liquid. Examples of the food product include foods and beverages. In addition to the above, sewage, river water, sea water, soil, hot spring water, bath water, tap water, a solubilized product thereof, an extract thereof, and the like may be used as the sample.

In the method of the present invention, an appropriate primer is used according to the type of the target nucleic acid and the type of the nucleic acid amplification method. The primer may contain conventionally known artificial nucleic acids such as bridged nucleic acid (BNA), phosphorothioate (PS)-oligo, peptide nucleic acid (PNA), morpholino oligos and 2'-O-substituted RNAs as long as extension of the 3' end by the nucleic acid amplification reaction is possible.

The nucleotide sequence of the primer may be a nucleotide sequence fully complementary to the nucleotide sequence of the region where the primer hybridizes to the target nucleic acid. Alternatively, the nucleotide sequence of the primer may contain a base that generates a mismatch site as long as the nucleotide sequence hybridizes with the target nucleic acid. In this case, the nucleotide sequence of the primer has, for example, 80% or more, preferably 90% or more, and more preferably 95% or more identity to the nucleotide sequence of the region where the primer hybridizes to the target nucleic acid.

As used herein, the term "hybridize" refers to that a whole or part of a predetermined polynucleotide forms a double strand via a hydrogen bond with a whole or part of another polynucleotide under stringent conditions. The term "stringent conditions" may be conditions commonly used by those skilled in the art under which hybridization of polynucleotides is performed. Examples thereof include conditions in which one polynucleotide molecule can specifically hybridize to the other polynucleotide molecule when there is at least 50%, preferably at least 75%, and more preferably at least 90% identity between the two polynucleotide molecules. Stringency of hybridization is known to be a function of temperature, salt concentration, base length and GC content of the oligonucleotide and concentration of a chaotropic agent contained in a hybridization buffer. As the stringent conditions, conditions described in, for example, Sambrook, J. et al., 1998, Molecular Cloning: A Laboratory Manual (2nd edition), Cold Spring Harbor Laboratory Press, New York.

As used herein, the term "nucleotide sequence fully complementary" refers to a nucleotide sequence that forms complementary base pairs of Watson-Crick model with all bases in the nucleotide sequence of a whole or part of a

predetermined polynucleotide. As used herein, the term "mismatch site" refers to a site in which complementary base pairs of Watson-Crick model cannot be formed because a base that is not complementary to a predetermined base in a nucleotide sequence of one polynucleotide molecule is present at a corresponding position in a nucleotide sequence of the other polynucleotide molecule when the two polynucleotide molecules are hybridized.

The polymerase of the present invention can be used in the same manner as the conventional Bst DNA polymerase. That is, the conditions of the amplification reaction in the method of the present invention can be equivalent to those of a nucleic acid amplification method using the conventional Bst DNA polymerase. Since the polymerase of the present invention has strand displacement activity, the polymerase of the present invention can perform the amplification reaction of the target nucleic acid under substantially isothermal conditions. The substantially isothermal conditions and the nucleic acid amplification method performed under the conditions are as described above.

When a quantitative amplification reaction such as a real-time LAMP method is performed, a fluorescent intercalator (for example, SYTO (trademark)-16, SYBR (trademark) Green, etc.), and a fluorescently labeled probe modified with a fluorescent substance at 5' end and with a quencher substance (for example, TaqMan (trademark) probe, etc.) at 3' end may be further added. The amplification product can be quantitatively detected by detecting fluorescence generated from the intercalator or probe incorporated into the amplification product. In the LAMP method, insoluble magnesium pyrophosphate is generated as a by-product as the amplification product increases. As the magnesium pyrophosphate increases, the reaction solution becomes cloudy. Therefore, the amount of the target nucleic acid can be calculated based on the measured value of turbidity or absorbance of the reaction solution or the time until these values reach a predetermined reference value.

Hereinbelow, the present invention will be described in detail by examples, but the present invention is not limited to these examples.

EXAMPLES

Example 1: Preparation of Recombinant Bst DNA Polymerase

A male pupa of Bombyx mori was inoculated with a recombinant baculovirus in which an expression construct encoding Bst DNA polymerase (SEQ ID NO: 3) was introduced with a DNA sequence encoding a His tag incorporated at the 5' side. The virus-inoculated pupa was stored at normal temperature for 7 days, and then the infected pupa was frozen at -70°C or lower. A grinding buffer (50 mM Tris-HCl, pH 7.5; 300 mM NaCl; 5 mM EDTA; 5 mM benzamidine hydrochloride; 0.5 mM PMSF; 0.5 mM DTT; 0.1% 1-phenyl-2-thiourea) was added to the frozen infected pupa, and the pupa was ground. The ground product was centrifuged at a low speed to collect the homogenate. The homogenate was heat-treated at 65°C for 20 minutes, followed by centrifuging to collect the supernatant. 50% Ammonium sulfate was added to the supernatant, and a precipitate was collected. The precipitate was dissolved in purification buffer (50 mM Tris-HCl, pH 7.5; 300 mM NaCl; 20 mM imidazole). CHAPS was added to the obtained solution to a final concentration of 1% to solubilize the solution. The solution was passed through a Ni sepharose column, and then a His-tag-fused Bst DNA polymerase derived from Bombyx mori (hereinafter, also referred to as "the polymerase of Example 1") was obtained by an elution buffer (50 mM Tris-HCl, pH 8.5; 300 mM NaCl; 250 mM imidazole). The solution containing the polymerase of Example 1 is an example of the reagent composition of the present invention.

Example 2: Sugar Chain Analysis

In general, it is known that a sugar chain is not added to a protein when it is prepared by an E. coli expression system. On the other hand, a protein prepared by a Bombyx mori baculovirus expression system can be modified with a sugar chain. Since the amino acid sequence of the polymerase of Example 1 (SEQ ID NO: 1) has a consensus sequence (Asn-Ile-Thr) for N-linked sugar chain modification at positions 437 to 439, a sugar chain can be added to the polymerase of Example 1. In Example 2, it is examined by sugar chain analysis whether a sugar chain was added to the polymerase of Example 1 and to a commercially available Bst DNA polymerase prepared by the E. coli expression system (Comparative Example). For the polymerase of Example 1, the N-linked sugar chain was released by a glycosidase that cleaves a bond between GlcNAc of the N-linked sugar chain and an aspartic acid residue, and the structure of the sugar chain was analyzed.

(1) Detection of presence or absence of sugar chain

For the polymerase of Example 1 and the polymerase of Comparative Example, the presence or absence of glycosylation was confirmed by lectin blot using concanavalin A (ConA). Specifically, a sample separated by SDS-PAGE was transferred to a PVDF membrane and treated with ConA-biotin at 4°C overnight. Thereafter, HRP-labeled streptavidin was added to perform enzyme labeling. DAB (3,3'-diaminobenzidine) was further added as a substrate of HRP to perform

an enzymatic reaction, and a product was detected. The results are shown in Fig. 3. In Fig. 3, lane 1 was a marker, lanes 2 to 7 were the polymerases of Example 1, lane 8 was the polymerase of Comparative Example (New England Biolabs), and lane 9 was calf small intestine-derived alkaline phosphatase (Oriental Yeast Co., Ltd.) (positive control). Lanes 2 to 4 were polymerases purified from a mixed male and female sample of the Bombyx mori pupa, and lanes 5 to 7 were polymerases purified from the Bombyx mori male pupa. As can be seen from Fig. 3, a sugar chain was detected in the polymerase of Example 1, and no sugar chain was detected in the polymerase of Comparative Example.

(2) Analysis of sugar chain structure

(2.1) Preparation of measurement sample

The polymerase of Example 1 was desalted and lyophilized, followed by redissolving in water (22.8 μL). A 5% solution (6 μL) of RapiGest (trademark) SF (Waters) was added to this solution, and the mixture was reacted at 90°C for 3 minutes, followed by allowing to stand at room temperature for 3 minutes. A solution (1.2 μL) of Rapid (trademark) PNGase F (New England Biolabs) was added to this solution, and the mixture was reacted at 50°C for 5 minutes, followed by allowing to stand at room temperature for 3 minutes. To this solution was added a sugar chain labeling reagent (12 μL) contained in GlycoWorks (trademark) RapiFluor-MS (trademark) N-Glycan kit (Waters), and the mixture was allowed to stand at room temperature for 5 minutes. To this solution was added acetonitrile (358 μL) for dilution. The RFMS-labeled glycans were solid-phase extracted using GlycoWorks HILIC μElution Plate (Waters). The extract was freeze-dried, followed by redissolving in a solvent to obtain a measurement sample.

(2.2) Measurement by liquid chromatography/mass spectrometry (LC/MS) method

The measurement sample (10 μL) obtained in the above 1) was subjected to LC measurement using a Waters H-Class system (Waters). The measurement was performed as follows. The measurement sample was added to ACQUITY UPLC (trademark) Glycan BEH Amide 130 A, 1.7 μm, 2.1 x 150 mm column equilibrated at a mobile phase (A: 50 mM ammonium formate solution (pH 4.4), B: 100% acetonitrile; A/B = 25/75) of 0.4 mL/min and a column temperature of 60.0°C. Thereafter, the measurement was performed under gradient conditions of A/B = 25/75 (0 min, 0.4 mL/min) - 46/54 (35 min, 0.4 mL/min) - 100/0 (36.5 min, 0.2 mL/min) - 100/0 (39.5 min, 0.2 mL/min) - 25/75 (43.1 min, 0.2 mL/min) - 25/75 (47.6 min, 0.4 mL/min) - 25/75 (55 min, 0.4 mL/min) at detection wavelengths of Ex. 265 nm and Em. 425 nm.

MS measurement was performed with Waters Xevo-G2XS QTOF (Waters). The MS measurement conditions were as follows: polarity: ES+, analysis unit: sensitivity mode, capillary voltage (kV): 3.00, sampling cone voltage (V): 80.0, source temperature (°C): 120, source offset voltage (V): 60, desolvation temperature (°C): 350, cone gas flow rate (L/Hr): 50.0, and desolvation gas flow rate (L/Hr): 800.0.

(3) Results

The analysis results are shown in Table 1. In the table, in the column of the estimated sugar chain structure, a circle is a mannose residue, a square is an N-acetyl-D-glucosamine residue, a triangle is a fucose residue, and an open circle is a glucose residue. In the table, "1" in the column of sugar chain type refers to a sugar chain represented by the following formula (1), "2" refers to a sugar chain represented by the following formula (2), "3" refers to a sugar chain represented by the following formula (3), "4" refers to a sugar chain represented by the following formula (4), and "5" refers to a sugar chain represented by the following formula (5). As can be seen from Table 1, the polymerase of Example 1 was shown to comprise multiple polymerases with varying sugar chains, i.e. any one of the sugar chains listed.

[Table 1]

| Time | Area | Area% | Estimated sugar chain structure | Sugar chain type | Measured m/z | Valence |
|---|---|---|---|---|---|---|
| 11.11 | 103812 | 59.0% | 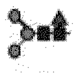 | 1 | 684.77 | 2 |
| 11.80 | 2034 | 1.2% | 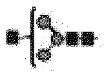 | 2 | 713.29 | 2 |
| 13.00 | 13188 | 7.5% | 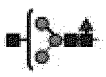 | 3 | 786.32 | 2 |

(continued)

| Time | Area | Area% | Estimated sugar chain structure | Sugar chain type | Measured m/z | Valence |
|---|---|---|---|---|---|---|
| 14.69 | 278 | 0.2% | 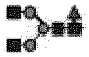 | 3 | 887.86 | 2 |
| 15.99 | 3649 | 2.1% | 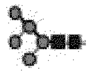 | 4 | 773.80 | 2 |
| 16.48 | 3935 | 2.2% | 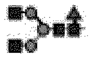 | 3 | 887.82 | 2 |
| 17.54 | 1729 | 1.0% | 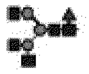 | 3 | 989.36 | 2 |
| 18.89 | 912 | 0.5% | 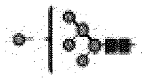 | 4 | 854.83 | 2 |
| 21.55 | 5139 | 2.9% | 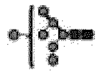 | 4 | 935,85 | 2 |
| 23.74 | 450 | 0.3% | 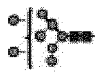 | 4 | 1016,88 | 2 |
| 24.00 | 15822 | 9.0% | 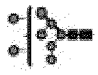 | 4 | 1016.88 | 2 |
| 25.57 | 284 | 0.2% | 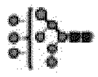 | 4 | 1097.90 | 2 |
| 25.80 | 12811 | 7.3% | 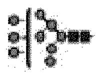 | 4 | 1097.90 | 2 |
| 27.51 | 536 | 0.3% | 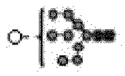 | 5 | 1178.92 | 2 |

[Chemical Formula 11]

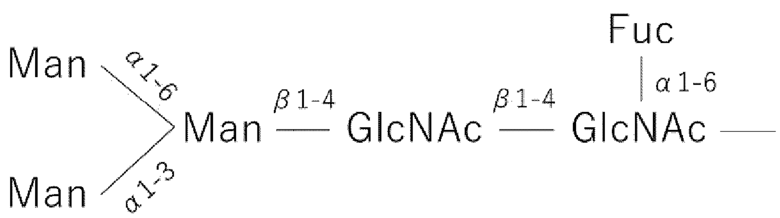

(1)

[Chemical Formula 12]

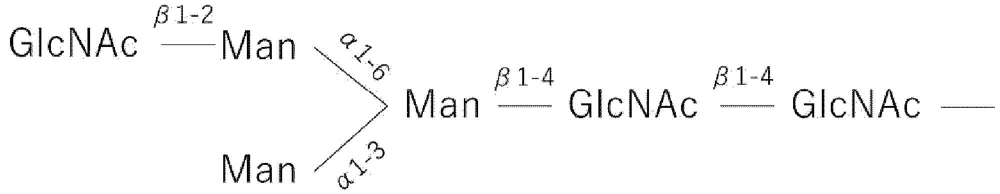

and/or

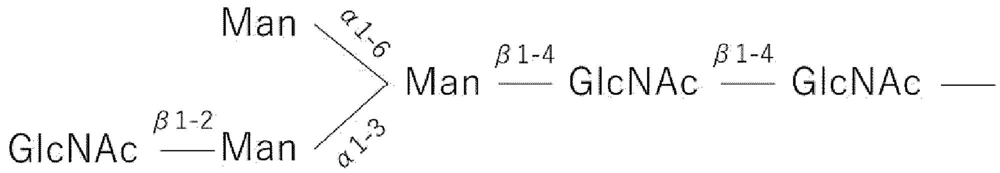

(2)

[Chemical Formula 13]

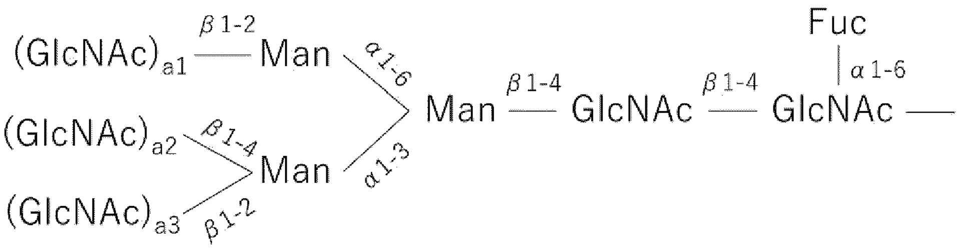

(3)

wherein a1, a2, and a3 are each independently an integer of 0 or more and 2 or less, and the sum of a1, a2, and a3 is 1 or more and 3 or less

[Chemical Formula 14]

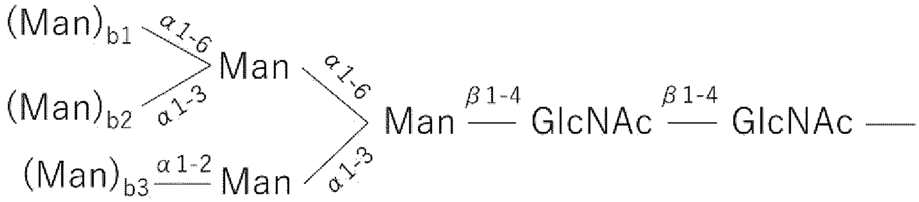

(4)

wherein b1, b2, and b3 are each independently an integer of 0 or more and 2 or less, and the sum of b1, b2, and b3 is 4 or less

[Chemical Formula 15]

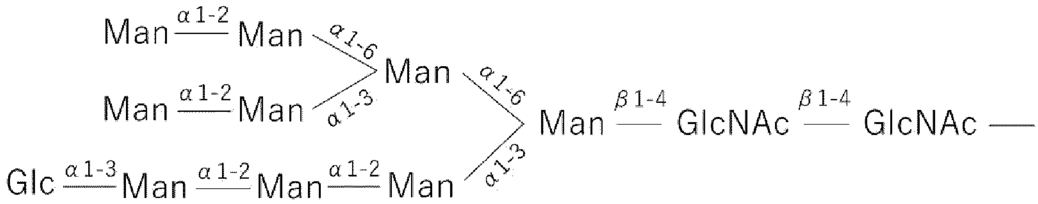

(5)

Example 3: Confirmation of Influence by Interfering Substances

The influence of the interfering substances on the nucleic acid amplification reaction using the polymerase of Example 1 was examined. Similarly, the influence of the interfering substances on the nucleic acid amplification reaction using the polymerase of Comparative Example was also examined. The interfering substances were substances known to cause inhibition of the nucleic acid amplification reaction, decrease in reaction rate, and the like.

(1) Preparation of template DNA sample

As a template DNA sample in the nucleic acid amplification reaction by Bst DNA polymerase, a sample containing human CK19 DNA was prepared as follows. First, PCR was performed using human CK19 plasmid DNA cloned with pBluescript (Agilent), Taq PCR master mix and RNase-free water contained in Taq PCR master mix kit (QIAGEN), and a primer set designed based on the base sequence of human CK19 to synthesize human CK19 DNA. The PCR product containing human CK19 DNA was purified with QIAquick PCR purification kit (QIAGEN) to obtain a human CK19 DNA solution. The DNA concentration of the obtained solution was calculated by absorbance measurement at 260 nm. Based on the calculated value, the copy number of human CK19 DNA in the solution was calculated. The human CK19 DNA solution was diluted with 50 ng/μL of yeast RNA (Thermo Fisher Scientific Inc.) so that the copy number of human CK19 DNA was 250,000. The obtained solution was used as a template DNA sample for subsequent experiments.

(2) Calculation of unit value of Bst DNA polymerase

In the polymerase of Comparative Example, the enzyme amount (unit value) and concentration based on the activity were known. The unit value of the polymerase of Example 1 was determined as follows, based on the measured value and the unit value of the nucleic acid amplification reaction using the polymerase of Comparative Example. First, the polymerase of Comparative Example was diluted with a glycerol solution (50 mM Tris-HCl, pH 7.5; 50% glycerol; 1 mM DTT; 50 mM KCl; 0.1 mM EDTA; 0.1% Triton (trademark) X-100) so as to have different concentrations from each other to prepare three enzyme solutions. Human CK19 DNA in the template DNA sample was measured using the enzyme solution at each concentration and a CK19 primer solution contained in gene amplification detection reagentLINOAMP (trademark) CK19 (Sysmex Corporation). For the measurement, a gene amplification detector RD-200 (Sysmex Corporation) was used. A calibration curve was made from the obtained measured value and the unit value of the polymerase of Comparative Example after dilution. The polymerase of Example 1 was diluted with a glycerol solution so that the calibration curve was within an applicable range, and human CK19 DNA in the template DNA sample was measured in the same manner as described above. The measurement results were applied to the calibration curve, and the unit value of the polymerase of Example 1 was determined. Then, the polymerase of Example 1 was diluted with a glycerol solution so as to correspond to 12 units to obtain an enzyme solution.

(3) Measurement by real-time LAMP (RT-LAMP) method

A reaction buffer containing the following six types of primers was prepared. The nucleotide sequence of each primer is shown below. The composition of the reaction buffer is shown in Table 2.

- FA Primer: 5'- ggagttctcaatggtggcaccaactactacacgaccatcca -3' (SEQ ID NO: 4)
- RA Primer: 5'- gtcctgcagatcgacaacgcctccgtctcaaacttggttcg -3' (SEQ ID NO: 5)
- F3 Primer: 5'- tggtaccagaagcagggg -3' (SEQ ID NO: 6)
- R3 Primer: 5'- gttgatgtcggcctccacg -3' (SEQ ID NO: 7)
- Loop primer F: 5'- agaatcttgtcccgcagg -3' (SEQ ID NO: 8)
- Loop primer R: 5'- cgtctggctgcagatga -3' (SEQ ID NO: 9)

[Table 2]

| Substance name | Concentration |
|---|---|
| TERGITOL (trademark) | 0.45% |
| Triton X-100 | 0.23% |
| DTT | 11.25 mM |
| Tris-HCl pH8.0 | 4.23 mM |
| Tris-HCl pH8.8 | 112.5 mM |

(continued)

| Substance name | Concentration |
|---|---|
| KCl | 11.25 mM |
| $(NH_4)_2SO_4$ | 22.5 mM |
| $MgSO_4$ | 16.11 mM |
| dNTP | 22.5 mM |
| FA Primer | 60.75 pmol |
| RA Primer | 60.75 pmol |
| F3 Primer | 3.825 pmol |
| R3 Primer | 3.825 pmol |
| Loop primer F | 45.75 pmol |
| Loop primer R | 45.75 pmol |
| Nuclease-free water | |

The reaction buffer (14 μL), 12 units of the polymerase (3 μL) of Comparative Example or the polymerase (3 μL) of Example 1 corresponding to 12 units, 50μM of fluorescent dye SYTO-16 (1 μL), 31250 copies of the template DNA sample (2 μL), and each of the interfering substances (5 μL) shown in Table 3 were mixed to prepare a reaction solution (25 μL). For comparison, nuclease-free water (5 μL) was added in place of the interfering substances to prepare a reaction solution containing no interfering substances. Each reaction solution was heated at 65°C for 1 hour to perform RT-LAMP. CFX96 (BioRad Laboratories, Inc.) was used as a measuring device. The increase in fluorescence intensity at 450 to 490 nm was measured with time by CFX96.

[Table 3]

| Interfering substances | Addition concentration |
|---|---|
| Urea | 125 mM |
| EDTA-2K | 1.5 mM |
| SDS | 0.01% |
| Glycerol | 12.5% |
| Dimethyl sulfoxide | 6.0% |
| Xylan | 10% |
| $Zn^{2+}$ | 0.09% |

(4) Results

Amplification curves were obtained from the measurement results for each of the polymerase of Comparative Example and the polymerase of Example 1. For each polymerase, Ct (threshold cycle) values, which are intersections of the amplification curve in the presence or absence of the interfering substances and a threshold of fluorescence intensity, were acquired. The threshold of fluorescence intensity was automatically set by CFX96. Using the Ct values of each polymerase in the presence or absence of the interfering substances, the difference in nucleic acid amplification change rate (ΔCt) between the polymerase of Comparative Example and the polymerase of Example 1 was calculated from the following formula.

[Expression 1]

$$\Delta Ct\ (\%) = \frac{Ct\ (E.coli\ w/\ ) - Ct\ (E.coli\ w/o)}{Ct\ (E.coli\ w/o)} \times 100 - \frac{Ct\ (Ex1\ w/\ ) - Ct\ (Ex1\ w/o)}{Ct\ (Ex1\ w/o)} \times 10($$

In the above formula, Ct (E. coli w/) was the Ct value of amplification by the polymerase of Comparative Example in the presence of the interfering substances. Ct (E. coli w/o) was the Ct value of amplification by the polymerase of Comparative Example in the absence of the interfering substances. Ct (Ex1 w/) was the Ct value of amplification by the polymerase of Example 1 in the presence of the interfering substances. Ct (Ex1 w/o) was the Ct value of amplification by the polymerase of Example 1 in the absence of the interfering substances. When the value of ΔCt (%) is positive, the

nucleic acid amplification change rate due to the influence of the interfering substances is lower in the polymerase of Example 1 than in the polymerase of Comparative Example. That is, it is shown that in the nucleic acid amplification reaction using the polymerase of Example 1, the influence of the interfering substances was reduced as compared with the case of using the polymerase of Comparative Example. Fig. 4 shows ΔCt for each of the interfering substances.

As can be seen from Fig. 4, even when any of urea, EDTA, SDS, glycerol, DMSO, xylan, and $Zn^{2+}$ was added, the influence of the interfering substances in the nucleic acid amplification reaction was reduced in the case of using the polymerase of Example 1 as compared with the case of using the polymerase of Comparative Example.

Example 4: Confirmation of Specificity of Reaction Temperature

The optimal reaction temperature of Bst DNA polymerase is said to be around 60 to 65°C. In general, in a nucleic acid amplification reaction, it is known that enzyme activity at a temperature lower than the optimal reaction temperature can cause non-specific nucleic acid amplification. RT-LAMP was performed at a temperature lower than 60°C using the polymerase of Example 1 to examine how much nucleic acid amplification occurs. Similarly, the polymerase of Comparative Example was also examined.

(1) Sample and reagents

The template DNA sample, the primers, and the reagents used for preparing the reaction solution were the same as those in Example 2. The reaction solution was prepared in the same manner as in the reaction solution containing no interfering substances in Example 2.

(2) Measurement by RT-LAMP method

The prepared reaction solution was heated at 58.0, 57.6, 56.7, 55.1, 53.2, 51.6, 50.5, 50.0, 48.8 or 47.4°C for 1 hour to perform RT-LAMP. Measurement was performed in the same manner as in Example 2 using CFX96 (BioRad Laboratories, Inc.) as a measuring device. For comparison, a nucleic acid amplification reaction was also performed at an optimal reaction temperature of 65°C.

(3) Results

Amplification curves were obtained from the measurement results for each of the polymerase of Comparative Example and the polymerase of Example 1. For each polymerase, Ct' values, which are intersections of the amplification curve and a threshold of fluorescence intensity, were acquired. The threshold of fluorescence intensity was automatically set by CFX96. Using the Ct' values of each polymerase, the nucleic acid amplification change rate (ΔCt') in the reaction temperature change of each polymerase was calculated from the following formula.

[Expression 2]

$$\Delta Ct'\ (\%) = \frac{Ct'\ (Temp.) - Ct'\ (65°C)}{Ct'\ (65°C)} \times 100$$

In the above formula, Ct' (Temp.) was the Ct' value of amplification by each polymerase at any reaction temperature. Ct' (65°C) was the Ct' value of amplification by each polymerase at an optimal reaction temperature of 65°C. That is, it is shown that a polymerase having a low value of the nucleic acid amplification change rate (ΔCt') at an arbitrary temperature suppresses a non-specific amplification reaction. The calculated value of ΔCt' (%) is shown in Fig. 5 as the nucleic acid amplification change rate. In the figure, "Example 1" refers to the polymerase of Example 1, and "Comparative Example" refers to the polymerase of Comparative Example. As can be seen from Fig. 5, at a temperature lower than 65°C, the Ct' value was lower in the case of using the polymerase of Example 1 than the case of using the polymerase of Comparative Example. That is, it was shown that at a temperature lower than 65°C, nucleic acid amplification was suppressed in the case of using the polymerase of Example 1 as compared with the case of using the polymerase of Comparative Example. For example, at 48.8°C, the difference in Ct' value between the polymerase of Example 1 and the polymerase of Comparative Example was about 10%. At 47.4°C, enzyme activity was still observed in the polymerase of Comparative Example, but no enzyme activity was observed in the polymerase of Example 1. As shown in Fig. 5, it was suggested that in the case of using the polymerase of Example 1, the amplification reaction at a temperature lower than the optimal reaction temperature was suppressed as compared with the case of using the polymerase of Comparative Example. For example, it is considered that when the reaction solution is heated, an unintended amplification

reaction that can generate a non-specific amplification product can be reduced before the reaction solution reaches the optimal reaction temperature.

## Claims

1. A Bst DNA polymerase comprising:

   (1) an amino acid sequence set forth in SEQ ID NO: 1; or
   (2) an amino acid sequence comprising a consensus sequence Asn-X-(Ser/Thr) wherein X is any amino acid residue other than a proline residue, and comprising an amino acid sequence having 80% or more identity to the amino acid sequence set forth in SEQ ID NO: 1, and having 5' to 3' DNA polymerase activity and strand displacement activity, wherein
   the consensus sequence comprises an N-linked sugar chain, and the N-linked sugar chain comprises three or more mannose residues.

2. The Bst DNA polymerase according to claim 1, wherein the N-linked sugar chain comprises nine or less mannose residues.

3. The Bst DNA polymerase according to claim 2, wherein the N-linked sugar chain has a structure represented by the following formula (I):

   [Chemical Formula 1]

   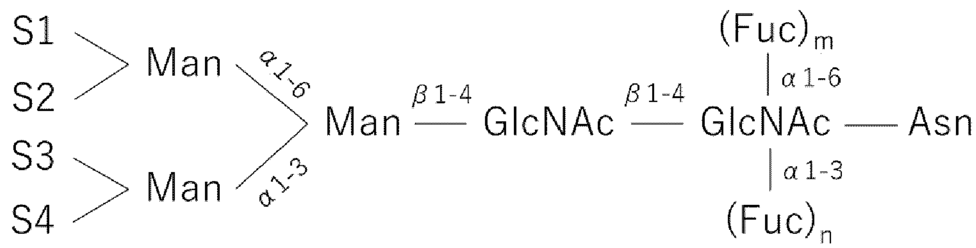

   (I)

   wherein Asn is an asparagine residue in the consensus sequence,

   Man is a mannose residue, GlcNAc is an N-acetyl D-glucosamine residue, Fuc is a fucose residue,
   m and n are each independently 0 or 1, and
   S1, S2, S3, and S4 are each independently absent or a sugar chain comprising one or more and five or less monosaccharide residues.

4. The Bst DNA polymerase according to claim 3, wherein the S1 comprises at least one monosaccharide residue selected from the group consisting of a mannose residue, an N-acetyl D-glucosamine residue, and a glucose residue.

5. The Bst DNA polymerase according to claim 3, wherein the S2 comprises at least one monosaccharide residue selected from the group consisting of a mannose residue, an N-acetyl D-glucosamine residue, and a glucose residue.

6. The Bst DNA polymerase according to claim 3, wherein the S3 comprises at least one monosaccharide residue selected from the group consisting of a mannose residue, an N-acetyl D-glucosamine residue, and a glucose residue.

7. The Bst DNA polymerase according to claim 3, wherein the S4 comprises at least one monosaccharide residue selected from the group consisting of a mannose residue, an N-acetyl D-glucosamine residue, and a glucose residue.

8. The Bst DNA polymerase according to claim 3, wherein the S1, the S2, the S3, and the S4 are absent.

9. The Bst DNA polymerase according to claim 3, wherein at least one sugar chain of the S1, the S2, the S3, and the S4 is an N-acetyl D-glucosamine residue.

10. The Bst DNA polymerase according to claim 3, wherein at least one sugar chain of the S1, the S2, the S3, and the S4

has a structure represented by the following formula (II):

[Chemical Formula 2]

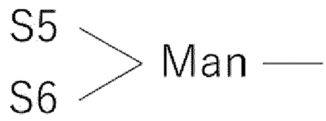

(II)

wherein S5 and S6 are each independently absent or a sugar chain comprising one or more and three or less mannose residues.

**11.** The Bst DNA polymerase according to claim 1, wherein at least one non-reducing end of the N-linked sugar chain is a mannose residue or an N-acetyl D-glucosamine residue.

**12.** The Bst DNA polymerase according to claim 8, wherein the m is 1 and the n is 0, or the m is 0 and the n is 1.

**13.** A reagent composition for a nucleic acid amplification method, comprising one or more of the Bst DNA polymerase according to any one of claims 1 to 12.

**14.** A nucleic acid amplification method comprising amplifying a target nucleic acid using the Bst DNA polymerase according to any one of claims 1 to 12 or the reagent according to claim 13.

FIG. 1

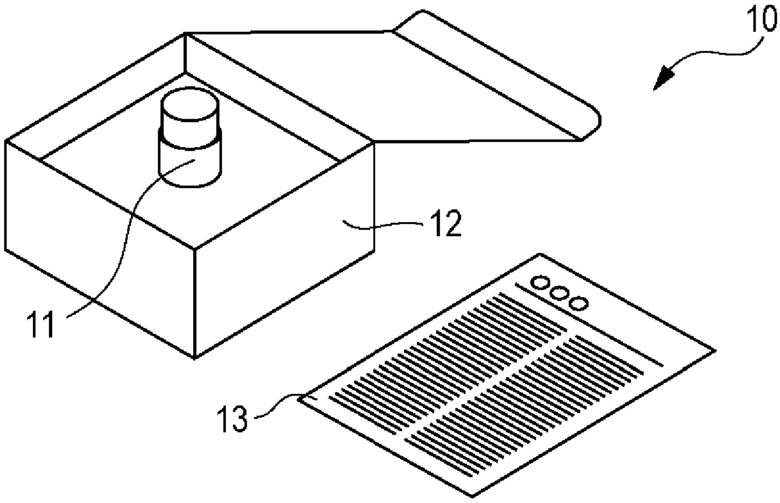


FIG. 2

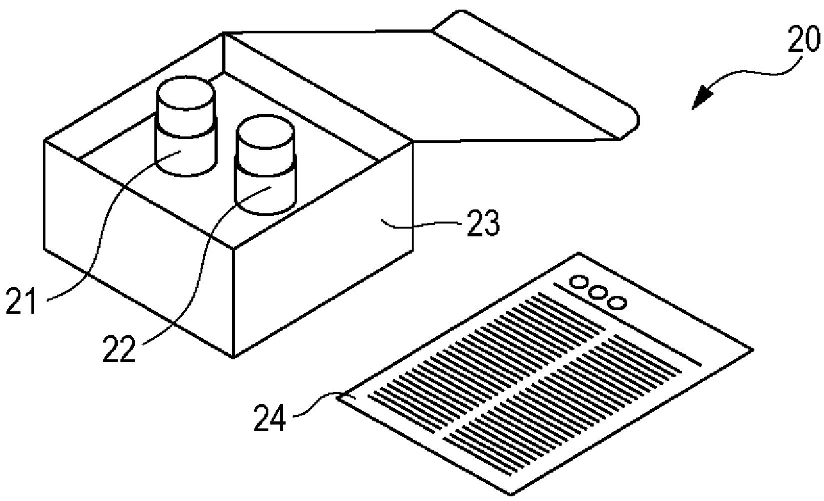

FIG. 3

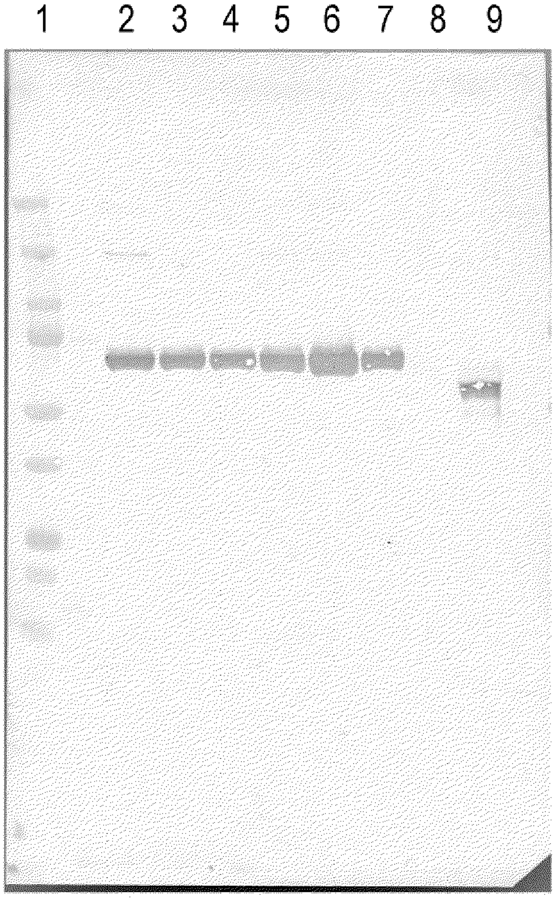

*FIG. 4*

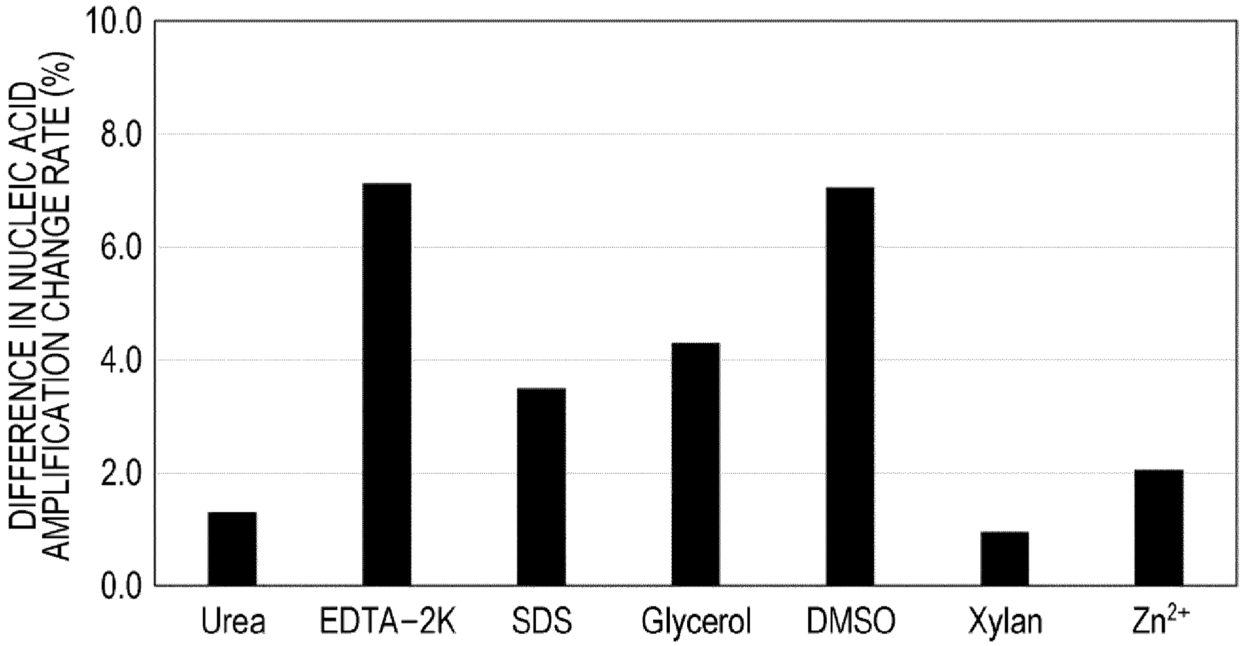
DIFFERENCE IN NUCLEIC ACID AMPLIFICATION CHANGE RATE (%)
10.0
8.0
6.0
4.0
2.0
0.0
Urea
EDTA-2K
SDS
Glycerol
DMSO
Xylan
Zn2+

*FIG. 5*

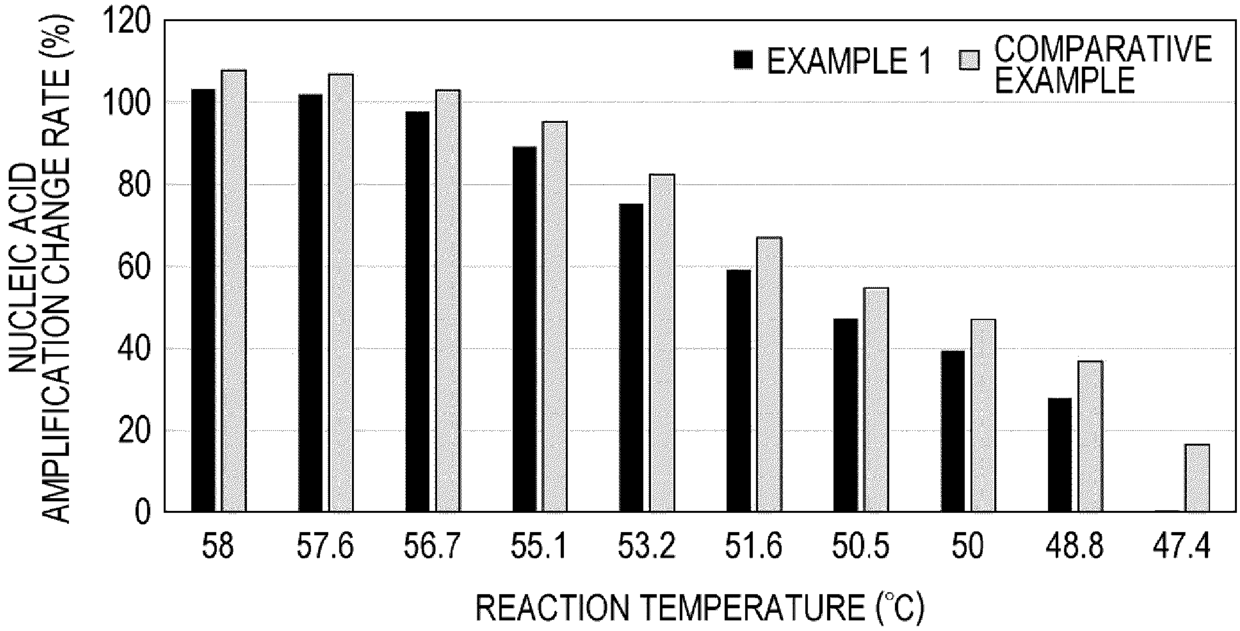
NUCLEIC ACID AMPLIFICATION CHANGE RATE (%)
120
100
80
60
40
20
0
EXAMPLE 1
COMPARATIVE EXAMPLE
58
57.6
56.7
55.1
53.2
51.6
50.5
50
48.8
47.4
REACTION TEMPERATURE (°C)

Europäisches Patentamt
European Patent Office
Office européen des brevets

# EUROPEAN SEARCH REPORT

Application Number
EP 24 19 2481

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2011/055737 A1 (NIPPON GENE CO LTD [JP]; OSHIMA TAIRO [JP] ET AL.) 12 May 2011 (2011-05-12) * paragraphs [0045] - [0049]; sequence 23 * ----- | 1-14 | INV. C12N9/12 |
| X | WO 2015/048763 A1 (LIFE TECHNOLOGIES CORP [US]) 2 April 2015 (2015-04-02) * paragraphs [0224], [0230]; sequence 16 * ----- | 1-14 | |

TECHNICAL FIELDS SEARCHED (IPC)

C12N

The present search report has been drawn up for all claims

1

| Place of search | Date of completion of the search | Examiner |
|---|---|---|
| Munich | 12 December 2024 | Petri, Bernhard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO.

EP 24 19 2481

This annex lists the patent family members relating to the patent documents cited in the above-mentioned European search report.
The members are as contained in the European Patent Office EDP file on
The European Patent Office is in no way liable for these particulars which are merely given for the purpose of information.

12-12-2024

| Patent document cited in search report | Publication date | Patent family member(s) | Publication date |
|---|---|---|---|
| WO 2011055737 A1 | 12-05-2011 | JP WO2011055737 A1 | 28-03-2013 |
| | | WO 2011055737 A1 | 12-05-2011 |
| WO 2015048763 A1 | 02-04-2015 | CN 105765082 A | 13-07-2016 |
| | | EP 3052652 A1 | 10-08-2016 |
| | | EP 4219739 A2 | 02-08-2023 |
| | | ES 2938585 T3 | 12-04-2023 |
| | | US 2015094211 A1 | 02-04-2015 |
| | | US 2017260512 A1 | 14-09-2017 |
| | | US 2020002688 A1 | 02-01-2020 |
| | | US 2020299655 A1 | 24-09-2020 |
| | | US 2021371834 A1 | 02-12-2021 |
| | | US 2024067941 A1 | 29-02-2024 |
| | | WO 2015048763 A1 | 02-04-2015 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5814506 A
- JP 2003052371 A
- JP H07303488 B
- US 6410278 B
- US 6291187 B
- US 5554517 A
- US 5270184 A
- US 20010053518
- EP 1167524 A


### Non-patent literature cited in the description


- **MA Y. et al.** *BioMed Research International*, 2016, vol. 2016, 2906484
- **PAIK I. et al.** *Biochemistry*, 2023, vol. 62, 410-418
- **SAMBROOK, J. et al.** Molecular Cloning: A Laboratory Manual. Cold Spring Harbor Laboratory Press, 1998